# EUROPEAN PATENT SPECIFICATION

(11) **EP 1 397 373 B1**
(45) Date of publication and mention of the grant of the patent: **16.02.2005**
(21) Application number: 02747245.5
(22) Date of filing: 07.06.2002
(51) Int. Cl.: C07H 13/04, C07H 9/04

(54) **IMPROVED SYNTHESIS OF ALLOFURANOSE**
VERBESSERTE SYNTHESEN VON ALLOFURANOSE
SYNTHESE AMELIOREE DE L'ALLOFURANOSE

(30) Priority: 07.06.2001 DK 200100888
(43) Date of publication of application: 17.03.2004
(73) Proprietor: Santaris Pharma A/S, 2970 Horsholm (DK)
(72) Inventor: KOCH, Troels, DK-2300 Copenhagen S (DK); HANSEN, Henrik, Frydenlund, DK-2610 Rodovre (DK)
(74) Representative: Plougmann & Vingtoft A/S
(86) International application number: PCT/DK2002/000385
(87) International publication number: WO 2002/098892

(56) References cited:
- EP-A- 0 379 397
- WO-A-00/56748
- CESAR M. / FUERTES R.: "Algunas s ntesis de d-alosa v a oxidaciones con dimetil sulf xido-anhidrido de ácido" BOLETIN DE LA SOCIEDAD QUIMICA DEL PERU, vol. 37, no. 4, 1971, pages 161-174, XP002902719
- W. SOWA ET AL: "The oxidation of 1,2;5,6-DI-O-isopropylidene-D-glucose by dimethyl sulfoxide-acetic anhydride." CANADIAN JOURNAL OF CHEMISTRY , vol. 44, 1966, pages 836-838, XP002902720

## Description

### FIELD OF THE INVENTION

The present invention relates to a new strategy for the synthesis of α-D-allofuranose, which is to be used for large-scale production of LNA (Locked Nucleic Acid) analogues with higher overall yields, and more cost efficient than previously known methods.

### BACKGROUND OF THE INVENTION

Synthesis of LNA (Locked Nucleic Acid) monomers were first reported by Wengel et al (Singh, S. K.; Nielsen, P., Koshkin, A. A. and Wengel, *J. Chem. Commun.,* **1998**, 455; Koshkin, A. A.; Singh, S. K.; Nielsen, P.; Rajwanshi, V. K.; Kumar, R.; Melgaard, M; Olsen, C. E. and Wengel, J., *Tetrahedron,* **1998**, 54, 3607). Depending on which monomer that is prepared, LNA monomer synthesis consists of 15-17 steps. Due to the length of the synthesis it is very important that all steps proceed in the most optimal way. The synthesis steps are optimised on four parameters:
1. Fast reaction time
2. Employing cheap reagents
3. Easy to handle
4. Proceeds in high overall yields

In the procedures cited in the art the starting material was 1,2:5,6-di-*O*-isopropylidene-α-D-allofuranose. 1,2:5,6-di-O-isopropylidene-α-D-allofuranose is commercially available (e.g. at Pfanstiel, CAS Number: 2595-05-03). Reducing the cost of the starting material by improving the synthesis is of great value for LNA synthesis.

1,2:5,6-di-O-isopropylidene-α-D-allofuranose is normally prepared from the much cheaper 1,2:5,6-di-O-isopropylidene-α-D-glucofuranose in two steps. The first step is oxidation of the secondary alcohol which is subsequently reduced to provide the allo-configuration. Oxidation of secondary hydroxyl groups to their corresponding carbonyl derivatives with dimethyl sulfoxide (DMSO) and acetic anhydride has been described (Albright J.D. and Goldman L., *J. Am*. *Chem*. *Soc.,* **1967**, 89:10). The oxidation of alcohols with acetic anhydride-DMSO is described to be a mild oxidative method and giving good yields with sterically hindered hydroxyl groups. They demonstrate in the paper that optimal oxidation is found in the case when ca. 20 times excess of acetic anhydride in relation to the alcohol, is used. All the experiments are performed on alkaloids and on steroids, thus no examples on furanoses are shown.

Also Horton D. and Jeweli J.S. (*Carbohydrate Res.,* **1966,** 2, 25i-260) and Horton, D. and Godman, J.L. (*Carbohydrate Res.,* **1968**, 6, 229-232) have used DMSO/Acetic anhydride but they point out that It is important to remove the reagents (DMSO/acetic anhydride) before further reactions and they use either evaporation at reduced pressure or lyophilation to remove the reagents. To carry out the reaction they use an excess of acetic anhydride between 7 and 26 fold.

Baker D. C. et al. (Baker D.C., Horton D. and Tindall C.G., *Carbohyd*. *Res.,* **1972**, 24 192-197) show that acetic anhydride/DMSO oxidation can be applied to 1,2:5,6-di-O-isopropylidene-α-D-glucofuranose and they underline that the method is not effective on large scale. For their large scale synthesis, 0.5 mole 1,2:5,6-di-*O*-isopropylidene-α-D-glucofuranose, they use a complex reaction mixture composed of chloroform, water, potassium metaperiodate, potassium carbonate, and the rare earth metal ruthenium dioxide. After a rather laborious work-up the hydrated ketone is isolated. Thus, they illustrate in this paper that it is not possible to mix the subsequent reduction step with the oxidation step. The overall yield of these two consecutive steps is 64 % of crude material. Furthermore, they claim that the DMSO/Acetic anhydride oxidation is not suitable for larger batches > 0.5 mole.

Sowa, W. and Thomas, G. H. S., (*Can. J. of Chem*., **1966**, Vol. 44, 836-838) used the DMSO/acetic anhydride oxidation of 1,2:5,6-di-O-isopropylidene-α-D-glucofuranose in small scale (10 mmole) using a 20 fold excess of acetic anhydride. The ulose was then reduced providing 1,2:5,6-di-O-isopropylidene-α-D-ailofuranose of poor quality, thus the product had to be column purified. Overall this procedure is not suitable for large-scale productions due to the large reagent consumption, two step procedure and the column purification step.

Youssefyeh R. D. et al. (Youssefyeh, R. D., Verhyden, J.P.H., Moffatt, J.G., *J. Org. Chem.* **1979**, 44(8), 1301-1309) employed the DMSO/Acetic anhydride oxidation and subsequent the reduction with sodium borohydride to prepare 1,2-*O*-isopropylidene-5-*O*-trityl-4-(trityloxymethyl)- α-D-*erythro*-pentofuranose from the corresponding *threo* derivative. Like Baker D. C. et al. they used a large excess of acetic anhydride (10 times) and performed the reaction in small scale (2.13 mmole).

Fuertes C. M. and Cesar M. (*Bol. Soc. quim. Peru,* **1972**. 37(4), 161-74,) prepared 1,2:5,6-di-O-isopropylidene-α-D-allofuranose by a consecutive two step reaction from 1,2:5,6-di-O-isopropylidene-α-D-glucofuranose. However, they used a 13 fold excess of acetic anhydride and allowed the oxidation to proceed for 72 h. The yield in the oxidation step was 60%. The subsequent reduction was performed in 75% yield, thus the overall yield in the sequential reactions was 45%.

### SUMMARY OF THE INVENTION

The present invention provides a novel strategy for the synthesis of allofuranose using glucofuranose as starting material in a one-pot reaction. The novel finding is that it is possible to carry out the oxidation of 1,2:5,6-di-O-isopropylidene-α-D-glucofuranose with DMSO/acetic anhydride and a reduction reaction in one pot obtaining high yield of recrystallised and analytically pure 1,2:5,6-di-O-isopropylidene-α-D-allofuranose.

The main advantages of the present invention comprise the following:
- One-pot reaction
- Better yield than previously reported two-step reactions
- Lower reagent consumption
- Easy work-up
- Ready to scale up

All the impurities are removed in a simple extraction step without using chromatography. Chromatography is not suitable for large-scale synthesis.

### DETAILED DESCRIPTION OF THE INVENTION

The surprising finding according the present invention is that it is possible to carry out the oxidation of 1,2:5,6-di-*O*-isopropylidene-α-D-glucofuranose with DMSO/acetic anhydride in one pot with the subsequent reduction of the 1,2:5,6-di-*O*-isopropylidene-α-D-ribofuranose-3-ulose (formula I). It is very surprising that the reduction can be carried out in the presence of the oxidation mixture. It is also surprising that it is possible to use this oxidation procedure in large scale (1 mole), and that the lowest reported amount of excess acetic anhydride (5 times) provides 58 % yield of recrystallised and analytical pure 1,2:5,6-di-O-isopropylidene-α-D-allofuranose.

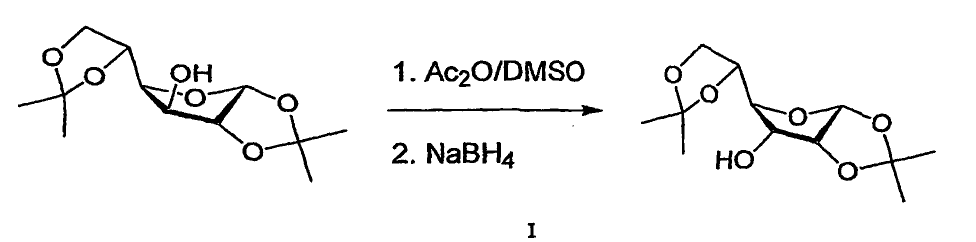

Thus, the combination of a facile one-pot reaction employing a minimum of reagent consumption makes the synthesis 1,2:5,6-di-*O*-isopropylidene-α-D-allofuranose much more cost effective. Also noteworthy is the fact that the work-up is very easy and that no column purification is needed to provide an analytical pure and crystalline product.

Although the invention has been developed based on the isopropylidene-protected sugars, it is believed that other protection groups of the acetal type may be applicable. Thus, the protective group used according to the present is typically an acetal such as Isopropylidene, cyclohexylidene or benzylidene. 1,2:5,6-di-*O*-isopropylidene-α-D-glucofuranose is commercially available and other protected glucofuranose derivatives are easily obtainable from glucose and the appropriate aldehyde/ketone using acid catalysis. The oxo compounds (aldehyde/ketone) may be used directly but may also be used as the corresponding alkylacetals/ketals. The acid catalysis may be effected by proton or Lewis acids as known in the art.

The oxidation reaction according to the present invention is preferably carried out using DMSO and an acid anhydride as an oxidising system. A preferred anhydride is acetic anhydride. The preferred molar ratio between 1,2:5,6-di-*O*-isopropylidene-α-D-glucofuranose and acetic anhydride is in the range of 1:3 and 1:9 and more preferably between 1:5 and 1:7. Other oxidising reagents such as N-halo-succinimides, chlorine, hypohalites, potassium metaperiodate-ruthenium dioxide, DMSO-*N,N'*-dicyclohexylcarbodiimide-pyridinium phosphate, DMSO-phosphorous pentaoxide, and chromium trioxide-pyridine complex may also be used.

In the present invention DMSO can be used as reagent and solvent but DMSO may also be used in combinations with other solvents so that the concentration of DMSO is reduced. Such solvents may not interfere with the oxidation/reduction reaction and would typically be polar non-protic solvents like NMP and DMF.

The oxidising reaction is typically be carried out at ambient temperature but the reaction can also be carried out at elevated temperatures. Typically temperatures in the range from 18-100°C preferable in the range from 20-70°C. The reaction will typically take from 1-48 h but the reaction time will preferably be in the range from 10-25 h.

The reduction reaction according to the present invention is typically performed using a reducing agent, typically a metal complex, that gives a high stereospecific yield of the allofuranose. Illustrative example of reducing agents are borohydrides for example sodium borohydride; borane; and aluminium hydrides such as lithium aluminium hydride and sodium bis (2-methoxyethoxy)aluminium hydride ("Vitride").

The reduction reaction is typically carried out at ambient temperature but the reaction can also be carried out eievated temperatures. Typically temperatures in the range from 18-70°C preferable in the range from 20-40°C. The reaction will typically take from 0.5-5 h but the reaction time will preferably be in the range from 1-3 h.

The yield of allofuranose obtained from the reaction according to the present invention, using glucofuranose as starting material, is in the range of 40%-90%, typically in the range of 50-70%.

In a preferred embodiment of the method according to the invention, 1,2:5,6-di-*O*-isopropylidene-α-D-glucofuranose is reacted with a mixture of DMSO and acetic anhydride in a molar ratio of 1:3 to 1:9 at 20-25°C, for around 12-48 h, typically for around 24 h, followed by reaction with sodium borohydride at 20-25°C, for around 0.5-2 h, typically for around 1 h, in order to yield 1,2:5,6-di-*O*-isopropylidene-α-D-allofuranose. The two reactions are performed sequentially, i.e. without isolation or purification of the Intermediate product, i.e. the ulose.

In one embodiment of the present invention the allofuranose is subsequently used in the synthesis of an LNA monomer, i.e. a conformationally locked nucleoside, e.g. as described in WO 99/14226 and subsequent WO 00/56746, WO 00/56748, WO 00/66604.

Thus, the method of the invention is particularly applicable for the production of an LNA monomer.

The invention has been described with reference to preferred embodiments thereof. However, it will be appreciated that those skilled in the art, upon consideration of this disclosure, may make modifications and improvements within the spirit and scope of the invention. The following non-limiting examples are illustrative of the invention.

### EXAMPLES

### Synthesis of 1,2:5,6-di-O-isopropylidene-α-D-allofuranose

1,2:5,6-di-*O*-isopropylidene-α-D-glucofuranose 260 g (1 mole) was stirred for 24 h in DMSO 2000 ml and Ac₂O 500 ml (5 mole) at RT. By that time, TLC (DCM (dichloromethane)/MeOH 95:5) Indicated complete reaction of the starting material. The solution was then cooled to 0 °C and sodium borohydride 16 g (0.42 mole) was added portion wise. By that time, TLC (DCM/MeOH 95:5) indicated complete formation of the target compound, approx. 1 h, water 2500 ml was added and the solution was extracted with DCM 3 x 1000 mL. The combined organic phases was washed with water 2 × 1000 ml and brine and dried with magnesium sulfate and evaporated to an oil. To the oil was added *tert*-butyl methylether (300 mL) and extracted with water 2 x 750 mL. The combined water phases were extracted with dichloroethane 2 x 400 mL. The combined organic phases was dried with magnesium sulfate and evaporated to an oil. Crystallisations twice from cydohexane, 500 ml and 300 ml respectively, gave 151 g (58 %) of pure 1,2:5,6-di-*O*-isopropylidene-a-D-allofuranose.
NMR (CDCl₃): 1.30 (s, 3, CH3), 1.32 (s, 3, CH3), 1.40 (s, 3, CH3), 1.51 (s, 3, CH3), 2.45 (d, 1, OH), 3.75 (q, 1, H-6), 4.00 (m, 3, H-4, H-5, H-7), 4.23 (m, 1, H-3), 4.55 (q, 1, H-2), 5.74 (m, 1, H-1)
- TLC spray:: Ketone - 100 mg DNPH, 90 mL EtOH, 10 ml konc. HCl Ketone and or product - 2 M H₂SO₄

## Claims

1. A method for the synthesis of 1,2:5,6 protected allofuranose wherein the starting material is 1,2:5,6 protected glucofuranose and the reaction is a one-pot reaction comprising an oxidation reaction and a reduction reaction wherein the reduction reaction is carried out in the presence of the oxidation mixture.

2. The method according to claim 1, wherein the 1,2 and 5,6 protection groups are of the acetal type.

3. The method according to claim 2, wherein the 1,2 and 5,6 protection groups are selected from the group consisting of isopropylidene, cyclohexylidene and benzylidene.

4. The method according to any of claims 1-3, wherein the 1,2:5,6 protected glucofuranose is 1,2:5,6-di-*O*-isopropylidene-α-D-glucofuranose.

5. The method according to any of claims 1-4, wherein the 1,2:5,6 protected allofuranose is 1,2:5,6-di-*O*-isopropylidene-α-D-allofuranose.

6. The method according to any of claims 1-5, wherein the oxidation is performed using DMSO and acid anhydride as an oxidising system.

7. The method according to claim 6, wherein the acid anhydride is acetic anhydride.

8. The method according to any of claims 1-7, wherein the reduction is performed using a metal hydride complex.

9. The method according to any of the claims 1-8, wherein the reduction is performed using a reducing agent selected from the group consisting of borohydride, boran-, and aluminium hydride.

10. The method according to claim 9, wherein the borohydride is sodium borohydride.

11. The method according to any of the preceding claims, wherein 1,2:5,6-di-*O*-isopropylidene-α-D-glucofuranose is reacted with a mixture of DMSO and acetic anhydride in a molar ratio of 1:3 to 1:9, followed by a reaction with sodium borohydride at 20-25 °C in order to yield 1,2:5,6-di-*O*-isopropylidene-α-D-allofuranose.

## Patentansprüche

1. Verfahren zum Herstellen von 1,2:5,6-geschützter Allofuranose, wobei das Ausgangsmaterial 1,2:5,6-geschützte Glucofuranose ist und die Reaktion eine Ein-Topf-Reaktion ist, umfassend eine Oxidationsreaktion und eine Reduktionsreaktion, wobei die Reduktionsreaktion in Gegenwart der Oxidationsmischung durchgeführt wird.

2. Verfahren nach Anspruch 1, wobei die 1,2- und 5,6-Schutzgruppen vom Acetal-Typ sind.

3. Verfahren nach Anspruch 2, wobei die 1,2- und 5,6-Schutzgruppen ausgewählt sind aus der Gruppe bestehend aus Isopropyliden, Cyclohexyliden und Benzyliden.

4. Verfahren nach einem der Ansprüche 1 bis 3, wobei die 1,2:5,6-geschützte Glucofuranose 1,2:5,6-di-O-Isopropyliden-α-D-Glucofuranose ist.

5. Verfahren nach einem der Ansprüche 1 bis 4, wobei die 1,2:5,6-geschützte Allofuranose 1,2:5,6-di-O-Isopropyliden-α-D-Allofuranose ist.

6. Verfahren nach einem der Ansprüche 1 bis 5, wobei die Oxidation ausgeführt wird unter Verwendung von DMSO und Säureanhydrid als ein oxidierendes System.

7. Verfahren nach Anspruch 6, wobei das Säureanhydrid Essigsäureanhydrid ist.

8. Verfahren nach einem der Ansprüche 1 bis 7, wobei die Reduktion ausgeführt wird unter Verwendung eines Metallhydrid-Komplexes.

9. Verfahren nach einem der Ansprüche 1 bis 8, wobei die Reduktion ausgeführt wird unter Verwendung eines Reduktionsmittels, das ausgewählt ist aus der Gruppe bestehend aus Borhydrid, Boran- und Aluminiumhydrid.

10. Verfahren nach Anspruch 9, wobei das Borhydrid Natriumborhydrid ist.

11. Verfahren nach einem der vorherigen Ansprüche, wobei 1,2:5,6-di-O-Isopropyliden-α-D-Glucofuranose reagiert wird mit einer Mischung aus DMSO und Essigsäureanhydrid in einem Molverhältnis von 1:3 bis 1:9, gefolgt von einer Reaktion mit Natriumborhydrid bei 20 bis 25 °C, um 1,2:5,6-di-O-Isopropyliden-α-D-Allofuranose zu ergeben.

## Revendications

1. Procédé de synthèse d'allofuranose 1,2:5,6 protégé dans lequel la matière première est le glucofuranose 1,2:5,6 protégé et la réaction est une réaction monotope comprenant une réaction d'oxydation et une réaction de réduction dans lequel la réaction de réduction est réalisée en présence du mélange d'oxydation.

2. Procédé selon la revendication 1, dans lequel les groupes de protection 1,2 et 5,6 sont du type acétal.

3. Procédé selon la revendication 2, dans lequel les groupes de protection 1,2 et 5,6 sont choisis dans le groupe consistant en l'isopropylidène, le cyclohexylidène et le benzylidène.

4. Procédé selon l'une quelconque des revendications 1 à 3, dans lequel le glucofuranose 1,2:5,6 protégé est le 1,2:5,6-di-*O*-isopropylidène-α-D-glucofuranose.

5. Procédé selon l'une quelconque des revendications 1 à 4, dans lequel l'allofuranose 1,2:5,6 protégé est le 1,2;5,6-di-*O*-isopropylidène-α-D-allofuranose.

6. Procédé selon l'une quelconque des revendications 1 à 5, dans lequel l'oxydation est réalisée avec du DMSO et de l'anhydride acide comme système oxydant.

7. Procédé selon la revendication 6, dans lequel l'anhydride acide est de l'anhydride acétique.

8. Procédé selon l'une quelconque des revendications 1 à 7, dans lequel la réduction est réalisée avec un complexe métallique hybride.

9. Procédé selon l'une quelconque des revendications 1 à 8, dans lequel la réduction est réalisée en utilisant un agent de réduction choisi dans le groupe consistant en le borohydrure, le borane et l'hydrure aluminium.

10. Procédé selon la revendication 9, dans lequel le borohydrure est le borohydrure de sodium.

11. Procédé selon l'une quelconque des revendications précédentes, dans lequel le 1,2:5,6-di-*O*-isopropylidène-α-D-glucofuranose est mis en réaction avec un mélange de DMSO et d'anhydride acétique selon un rapport molaire de 1:3 à 1:9, suivi d'une réaction avec le borohydrure de sodium à 20-25 °C afin de donner le 1,2:5,6-di-*O*-isopropylidène-α-D-allofuranose.
